# EUROPEAN PATENT SPECIFICATION

(11) **EP 2 023 961 B1**
(45) Date of publication and mention of the grant of the patent: **24.02.2016**
(21) Application number: 07729191.2
(22) Date of filing: 16.05.2007
(51) Int. Cl.: C12N 15/10, A61K 48/00, C12N 5/00, C12N 15/63

(54) **METHOD FOR ISOLATING HOMOGENEOUS POPULATIONS OF TRANSDUCED PROGENITORS STABLY EXPRESSING SPECIFIC LEVELS OF A TRANSGENE**
VERFAHREN ZUR ISOLIERUNG VON HOMOGENEN POPULATIONEN VON UMGEWANDELTEN VORLÄUFERN MIT STABILER EXPRESSION VON SPEZIFISCHEN STUFEN EINES TRANSGENS
PROCÉDÉ PERMETTANT D'ISOLER DES POPULATIONS HOMOGÈNES DE PROGÉNITEURS TRANSDUITS EXPRIMANT DE MANIÈRE STABLE DES NIVEAUX SPÉCIFIQUES D'UN TRANSGÈNE

(30) Priority: 17.05.2006 US 747426 P
(43) Date of publication of application: 18.02.2009
(73) Proprietor: Banfi, Andrea, 4056 Basel (CH); Misteli, Heidi, 4054 Basel (CH)
(72) Inventor: Banfi, Andrea, 4056 Basel (CH); Misteli, Heidi, 4054 Basel (CH)
(74) Representative: Latscha Schöllhorn Partner AG
(86) International application number: PCT/EP2007/054743
(87) International publication number: WO 2007/132012

(56) References cited:
- WO-A2-2004/044142
- OZAWA CLARE R ET AL: "Microenvironmental VEGF concentration, not total dose, determines a threshold between normal and aberrant angiogenesis." JOURNAL OF CLINICAL INVESTIGATION, vol. 113, no. 4, February 2004 (2004-02), pages 516-527, XP002445039 ISSN: 0021-9738 cited in the application
- VON DEGENFELD GEORGES ET AL: "Myoblast-mediated gene transfer for therapeutic angiogenesis and arteriogenesis." BRITISH JOURNAL OF PHARMACOLOGY, vol. 140, no. 4, October 2003 (2003-10), pages 620-626, XP002445040 ISSN: 0007-1188
- BANFI ANDREA ET AL: "Critical role of microenvironmental factors in angiogenesis." CURRENT ATHEROSCLEROSIS REPORTS MAY 2005, vol. 7, no. 3, May 2005 (2005-05), pages 227-234, XP009087649 ISSN: 1523-3804
- BANFI ANDREA ET AL: "Manipulation of microenvironmental VEGF determines a dose-dependent transition between normal and aberrant angiogenesis in vivo." TISSUE ENGINEERING, vol. 9, no. 4, August 2003 (2003-08), pages 793-794, XP009087657 & SECOND MEETING OF THE EUROPEAN TISSUE ENGINEERING SOCIETY; GENOA, ITALY; SEPTEMBER 03-06, 2003 ISSN: 1076-3279
- CHENG L ET AL: "Sustained gene expression in retrovirally transduced, engrafting human hematopoietic stem cells and their lympho-myeloid progeny" BLOOD, W.B. SAUNDERS, PHILADELPHIA, VA, US, vol. 92, no. 1, 1 July 1998 (1998-07-01), pages 83-92, XP002112126 ISSN: 0006-4971
- VON DEGENFELD GEORGES ET AL: "Microenvironmental VEGF distribution is critical for stable and functional vessel growth in ischemia" FASEB JOURNAL, vol. 20, no. 14, December 2006 (2006-12), XP002445043 ISSN: 0892-6638
- MISTELI ET AL.: "CONTROLLED ANGIOGENESIS BY FACS-PURIFIED ENGINEERED MYOBLAST POPULATIONS WHICH STABLY EXPRESS HOMOGENEOUS SPECIFIC VEGF LEVELS" EUROPEAN SURGICAL RESEARCH, vol. 38, no. 1, 17 May 2006 (2006-05-17), pages 1-1, XP002445063
- MANCIA F ET AL: "Optimization of Protein Production in Mammalian Cells with a Coexpressed Fluorescent Marker", STRUCTURE, CURRENT BIOLOGY LTD., PHILADELPHIA, PA, US, vol. 12, no. 8, 1 August 2004 (2004-08-01) , pages 1355-1360, XP025917223, ISSN: 0969-2126, DOI: 10.1016/J.STR.2004.06.012 [retrieved on 2004-08-05]
- ROYER YOHAN ET AL: "High-throughput gateway bicistronic retroviral vectors for stable expression in mammalian cells: Exploring the biologic effects of STAT5 overexpression", DNA AND CELL BIOLOGY, vol. 23, no. 6, June 2004 (2004-06), pages 355-365, ISSN: 1044-5498
- SU LISHAN ET AL: "Hematopoietic stem cell-based gene therapy for acquired immunodeficiency syndrome: Efficient transduction and expression of RevM10 in myeloid cells in vivo and in vitro", BLOOD, AMERICAN SOCIETY OF HEMATOLOGY, US, vol. 89, no. 7, 1 January 1997 (1997-01-01), pages 2283-2290, XP002468028, ISSN: 0006-4971
- Rafael ET AL: "Gene therapy for the treatment of AIDS: animal models and human clinical experience", Frontiers in Bioscience, vol. 4, no. 1-3, 15 March 1999 (1999-03-15), pages d468-475, XP055099427, ISSN: 1093-9946, DOI: 10.2741/Amado
- LIU XUEDONG ET AL: "Generation of mammalian cells stably expressing multiple genes at predetermined levels", ANALYTICAL BIOCHEMISTRY, ACADEMIC PRESS INC, NEW YORK, vol. 280, no. 1, 10 April 2000 (2000-04-10), pages 20-28, XP002187203, ISSN: 0003-2697, DOI: 10.1006/ABIO.2000.4478

## Description

### Field of the invention

The invention relates to a method for rapidly isolating homogeneous populations of transduced progenitors stably expressing specific levels of a transgene by FACS, useful for cell-based gene therapy.

### Background of the invention

Vascular Endothelial Growth Factor (VEGF) is an attractive candidate being investigated to achieve therapeutic angiogenesis in ischemic diseases, but it also can induce aberrant vasculature and angioma growth, which can call into question its therapeutic utility (M.L. Springer et al., Mol. Cell 2:549-558,1998; P. Carmeliet, Nat. Med. 6:1102-1103, 2000; S. Ylä-Herttuala et al., J. Am. Coll. Cardiol. 49:1015-1026, 2007). It was recently shown that a discrete threshold in VEGF dosage exists, below which normal stable capillaries are induced and above which angioma growth occurs. However, to predictably avoid toxic effects, it is necessary to ensure homogeneous expression in the microenvironment around each transduced muscle fiber (C.R. Ozawa, A. Banfi et al., J. Clin. Invest. 113:515-527, 2004). In fact, since VEGF remains tightly localized in tissue around the cells producing it, different growth factor concentrations do not average each other, even between neighbouring muscle fibers. Therefore, a few "hotspots" of high expression are sufficient to cause hemangioma growth even if the total VEGF dose is rather low. This finding helps to explain the apparent difficulty to achieve a manageable therapeutic window in clinical trials of VEGF gene therapy (A. Banfi et al., Curr. Atheroscl. Rep. 7:227-234, 2005). In fact, currently employed gene therapy methods, such as direct injection of constitutive adenoviral and plasmid vectors, only allow control of the total dose (titer) of gene delivered, but not of the distribution of microenvironmental levels achieved in vivo. Therefore, in order to avoid even rare "hotspots" of expression, the total dose must be kept low and efficacy is wasted.

Lishan Su et al. (Blood 89:2283-2290, 1997) describe a hematopoietic stem cell-based gene therapy using an amphotropic retroviral vector encoding a bicistronic gene coexpressing the desired protein and the murine CD8α' chain (lyt2). A review of gene therapy for the treatment of AIDS (animal models and human clinical experience) is available as published by R.G. Amado et al., Frontiers in Bioscience 4:468-475, 1999.

Xuedong Liu et al. (Anal. Biochem. 280:20-28, 2000) describe the generation of mammalian cells stably expressing multiple genes at predetermined levels, using retroviral vectors and genes linked by an internal ribosomal entry site (IRES) with a selectable marker. F. Mancia et al. (Structure 12:1355-1360, 2004) describe a procedure for the direct selection of stable mammalian cell lines expressing a marker protein linked to the desired protein through an IRES. Y. Royer et al. (DNA and Cell Biology 23:355-365, 2004) describe high-throughput "gateway" bicistronic retroviral vectors for stable expression in mammalian cells based on the use of an IRES allowing simultaneous expression of a protein of interest and a fluorescence marker.

WO2004/044142A2 describes modified mesenchymal stem cells and methods of treating injury and disease using such cells.

### Summary of the invention

The present invention describes a method for producing precursor cells expressing a target gene at desired levels, such as efficacious levels, more efficacious levels or microenvironmentally sub-toxic levels, comprising
(a) providing a heterogeneous population of cultured precursor cells;
(b) stably transducing said precursor cells with (i) a target gene linked to (ii) a cell-surface marker gene via a sequence accommodating translation of (i) and (ii) from the same mRNA;
(c) correlating an expression level of the target gene with that of the cell-surface marker gene;
(d) selecting a reference clone of cultured precursor cells expressing said gene at a threshold of desirability, e.g., at a threshold of (i) efficacious expression, (ii) increased efficacious expression or (iii) microenvironmentally sub-toxic expression of said gene; and
(e) isolating precursor cells having the (i) same or lower levels or (ii) the same or higher levels of expression of said target gene than said reference clone via said cell-surface marker.

The scope of the invention is defined by the appended claims.

In a variant of this method, two reference clones are selected in step (d) expressing said gene at a lower threshold and at an upper threshold, respectively, and precursor cells are isolated in step (e) having a level of expression of said target gene between the levels of expression of the two reference clones. In a further variant, two or more, e.g., three, four or five, reference clones are selected in step (d) expressing said gene at different levels, the plurality of clones used to define a lower and an upper threshold of target gene expression, and precursor cells isolated in step (e) having a level of expression of said target gene at any desired level between the lower and higher threshold levels of expression.

The expression level of said cell-surface marker in (d) may be quantified via Fluorescence Activated Cell Sorting (FACS). The expression level of said target gene in (d) may be quantified via methods such as, but not limited to, ELISA, RIA, EIA, Western Blot or Surface Plasmon Resonance.

The target gene may be a Vascular Endothelial Growth Factor (VEGF) or any other gene, such as, but not limited to, a gene encoding a transcription factor or a growth factor receptor, having a toxic effect when expressed in a host microenvironment in excess of a determined threshold level or for which a minimum expression level is desired for efficacy or for increased efficacy, such as, but not limited to, constitutively active HIF-1 alpha, PDGFR beta or PDGFb.

Said cell-surface marker may be any endogenous cell-surface compound readily quantifiable, preferably in truncated form without the original biological activity, for example, a truncated version of CD8/CD8a.

The sequence accommodating translation of (b)(i) and (b)(ii) from the same mRNA may be an internal ribosomal entry site (IRES).

Said precursor cells may be primary myoblasts, bone marrow derived mesenchymal stem cells, adipose tissue-derived mesenchymal stem cells, or cardiac stem cells.

The invention is also directed towards a system for producing precursor cells expressing a target gene in a host at microenvironmentally desirable levels, e.g., sub-toxic levels comprising
(a) a construct integrating into the host genome, such as, but not limited to, a retroviral construct, providing (i) a target gene linked to (ii) a cell-surface marker via a sequence accommodating translation of (i) and (ii) from the same mRNA; and
(b) instructions for:
   (1) stably transducing precursor cells with said construct; and
   (2) correlating expression levels of the target gene with that of the cell-surface marker so that expression levels of said target gene can be assessed in said precursor cells via said cell surface marker.

The invention is also directed towards a system for in vivo gene therapy comprising a homogenous population of precursor cells stably transduced with a construct providing (i) a target gene linked to (ii) a cell-surface marker via a sequence accommodating translation of (i) and (ii) from the same mRNA, wherein the homogenous population has been selected via said cell surface marker so that the expression levels of said target gene are in the desired range, e.g., efficacious, more efficacious or microenvironmentally sub-toxic, in a host microenvironment into which said precursor cells are to be introduced.

The invention is also directed towards a controlled level expression gene therapy comprising
(a) obtaining a heterogeneous population of precursor cells from a patient;
(b) culturing and stably transducing said precursor cells with (i) a target gene linked to (ii) a cell-surface marker via a sequence accommodating translation of (i) and (ii) from the same mRNA;
(c) correlating in clones of said precursor cells expression levels of the target gene with that of the cell-surface marker gene;
(d) selecting, as a reference clone, a clone of (c) expressing said target gene at a level that defines a threshold of desirability for said patient, e.g., of (i) sub-toxicity or (ii) a higher level of efficacy;
(e) isolating via said cell-surface marker cells having the (i) same or lower levels or (ii) the same or higher levels of expression of said target gene than said reference clone; and
(f) reintroducing the cells of (e) into said patient.

In a variant of this method, two reference clones are selected in step (d) expressing said gene at a lower threshold and at an upper threshold, respectively, and precursor cells are isolated in step (e) having a level of expression of said target gene between the levels of expression of the two reference clones.

Furthermore, in a controlled level expression gene therapy said steps (c) and (d) may be omitted and the information obtained in steps (c) and (d) from the application of the method on precursor cells of another patient be used in place thereof, i.e. the correlation of expression levels of the target gene with that of the cell-surface marker gene and the selection of a reference clone.

### Brief description of the figures

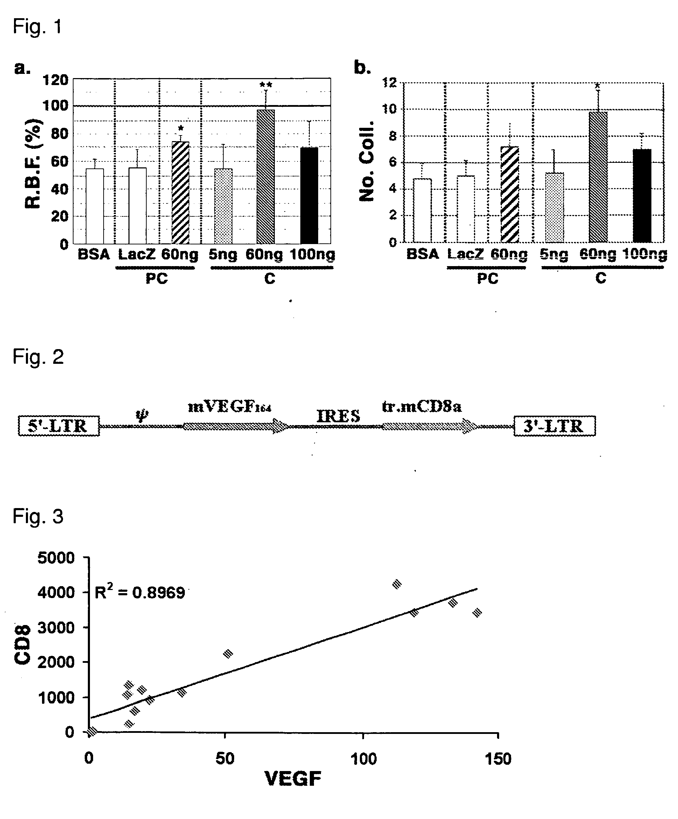

*Figure 1*. Control over microenvironmental distribution enhances the efficacy of VEGF gene delivery in ischemic muscle.
   Vehicle only (BSA), control cells (LacZ) or myoblast populations expressing the indicated VEGF levels (5, 60 or 100 ng/10⁶ cells/day) were implanted in ischemic mouse hind limbs. Analysis after 2 weeks shows that homogeneous distribution of expression levels in the microenvironment, achieved by a clonal population (C), significantly improves both relative blood flow (a.) and number of collateral arteries (b.) compared to the same total dose (60 ng/10⁶ cells/day) delivered as the average of dishomogeneous levels by a polyclonal population (PC).
   R.B.F. (%) = Relative blood flow (% of non-ischemic). No. Coll. = Number of collaterals. PC = polyclonal, C = clonal.
*Figure 2*. Structure of the VICD8 retroviral construct.
   Expression of the mouse VEGF₁₆₄ gene (mVEGF₁₆₄) was linked to that of the FACS-sortable cell-surface marker truncated CD8a (tr. mCD8a) through an IRES sequence. This cassette was inserted into a retroviral construct, so that stable expression of the two genes is achieved after integration into the genome of the target cell.
   LTR = retroviral long terminal repeats; ψ = retroviral packaging signal.
*Figure 3**.* Correlation of VEGF production with amount of fluorescence in standard populations.
   Individual clones homogeneously expressing different levels of VEGF were analyzed on the FACS after staining with a fluorescently-labeled antibody against mCD8a. The intensity of CD8 staining and the production of VEGF (measured by ELISA) were found to correlate with a value of R² = 0.8969.
   X-axis: VEGF production (ng/10⁶ cells/day), Y-axis: CD8 expression (fluorescence intensity units).

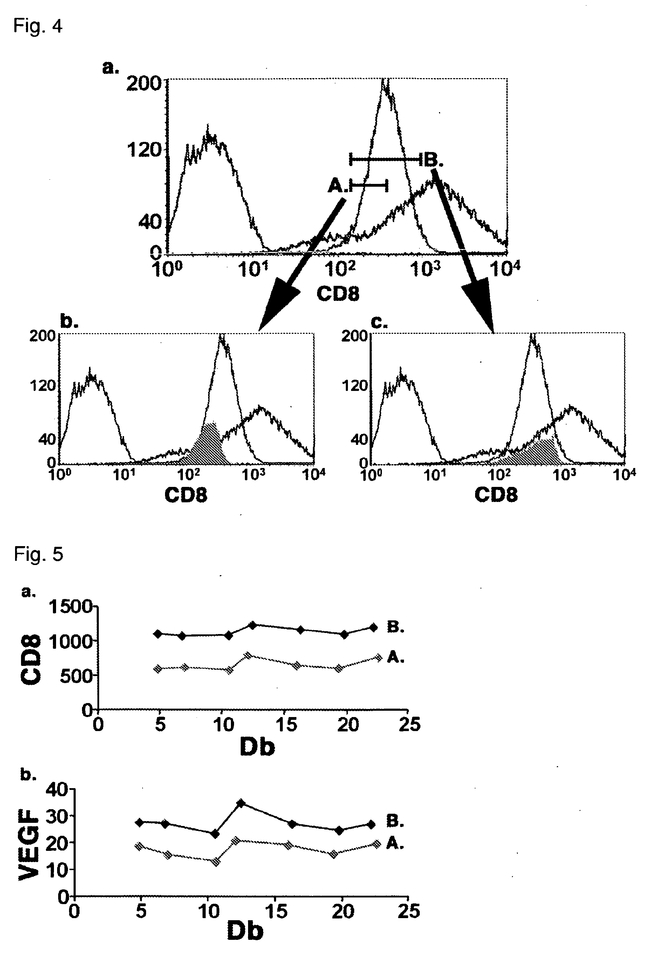

*Figure 4**.* FACS-sorting of desired populations based on CD8 expression level.
   Almost all cells in the primary transduced population express CD8 (and VEGF) compared to the negative control (below 2x10¹), but the spectrum of expression levels is widely heterogeneous (between 2x10¹ and 10⁴). Based on the range of fluorescence detected in the reference clone (curve between 10² and 10³ reaching 200 counts), two different polyclonal sub-populations have been sorted, expressing lower (A.) or higher (B.) CD8 levels. The purity of the sorted populations was determined by re-analysis on the FACS (dark plots in panels b. and c.) and they were found to be completely contained within the respective sorting gates.
   X-axis: CD8 expression (fluorescence intensity units), Y-axis: cell counts.
*Figure 5**.* Stability of expression of CD8 and of VEGF in sorted populations during in vitro expansion.
   The sorted populations described in Fig. 4 (A. and B.) were expanded for about 25 population doublings in vitro. The expression of CD8a (measured by FACS, panel a.) and of VEGF (measured by ELISA, panel b.) was found to be stable, with similar values immediately after sorting and after about 1x10⁷-fold expansion (23 doublings).
   Db = population doublings; CD8 = CD8 expression (fluorescence intensity units); VEGF = VEGF production (ng/10⁶ cells/day)

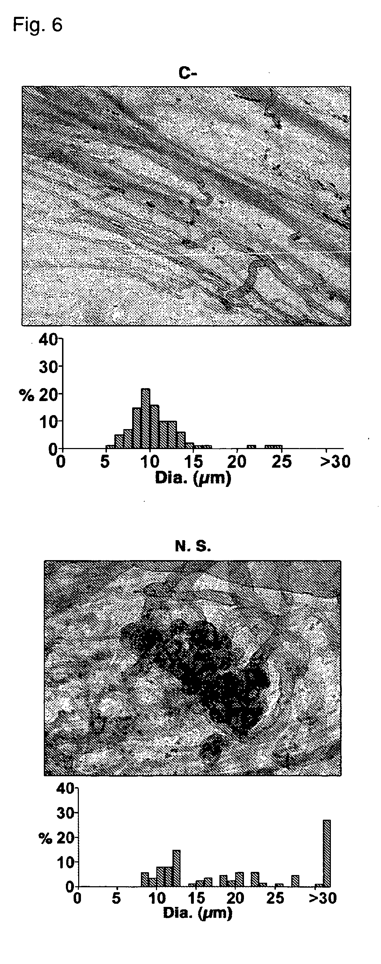

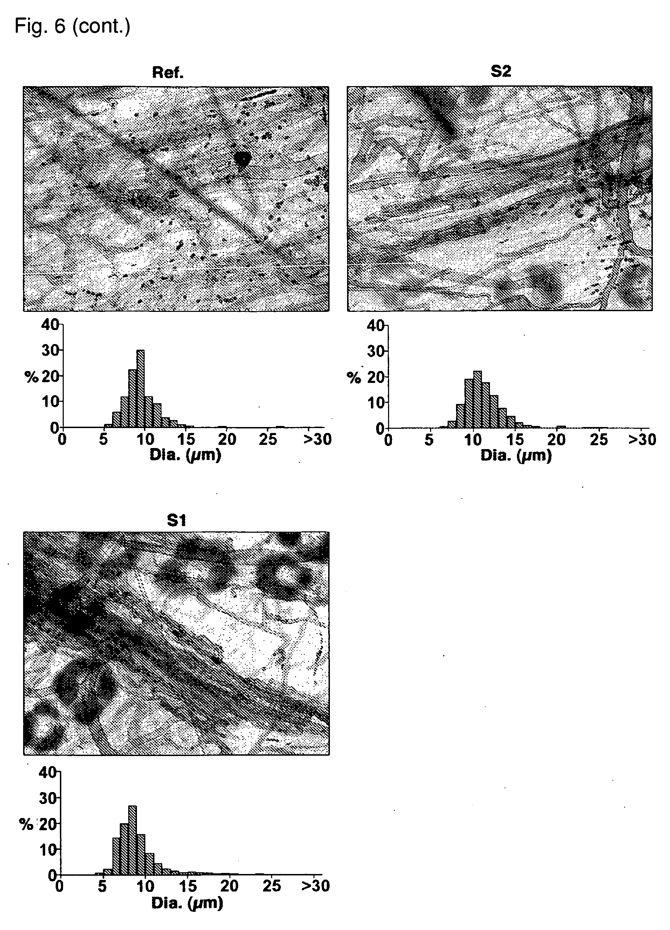

*Figure 6**.* In vivo angiogenesis after implantation of purified VEGF-expressing myoblasts. Cells isolated from a heterogeneous population based on their CD8 expression (A. and B. gates in Fig. 4a.) induce normal angiogenesis (S1 and S2), similarly to the clone used as a reference to sort them (Ref.), but bypassing the cloning step. The unsorted population (N.S.) causes the widespread growth of hemangiomas instead. In the top panels vasculature was stained with lectin and implanted myoblasts were stained for LacZ expression using the X-gal reaction. In the lower panels, the vessel diameter (Dia.(µm)) distribution was quantified in the implantation areas and is expressed as the percentage of vessels (%) with a given diameter in micrometers (µm), rounded to the nearest micrometer.
   C- = control cells; N.S. = not sorted population; Ref. = reference clone;
   S1 = sorted with gate A. (see Fig. 4); S2 = sorted with gate B. (see Fig. 4)

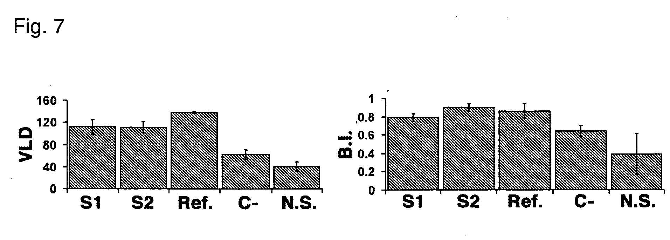

*Figure 7*. Morphological characterization of induced angiogenesis.
   Rapidly purified populations induce a doubling of the vessel length density (VLD) and an increase of the branching index (B.I.) relative to controls, similarly to the reference clone, whereas the unsorted population actually reduces the number of vessels in the implantation areas.
   VLD = Vessel Length Density (mm of vessel length/mm²); B.I. = Branching Index (number of branch points/100 µm of vessel length);
   C- = control cells; N. S. = not sorted population; Ref. = reference clone;
   S1 = sorted with gate A.; S2 = sorted with gate B.

### Detailed description of the invention

The terms used hereinabove and hereinbelow have the following meaning:
*Precursor cells* - are cells capable of giving rise to a differentiated progeny in vivo, after in vitro expansion and/or genetic modification. Examples include myoblasts and muscle satellite cells, cardiac progenitors, bone-marrow or adipose tissue-derived mesenchymal progenitor cells (also called mesenchymal stem cells), multipotent adult progenitor cells (MAPC), neural stem cells, epidermal stem cells, corneal stem cells and conjunctival stem cells.

*Microenvironmental* - refers to the level of expression of a transgene in each individual transduced cell, which determines the amount of gene product that accumulates in the microenvironment around it in vivo, as opposed to the average expression by the total population. The two expressions (total and microenvironmental) coincide when the population is the progeny of a single clone, in which every cell produces the same level.

*Sub-toxic* - refers to no toxicity or very low toxicity, not causing any persistent damage. In the context of target genes supporting angiogenesis sub-toxic is defined as inducing non-aberrant angiogenesis. This may further be defined as the growth of new blood vessels with little or no detectable formation of defective vascular structures, which differ in structure and/or function from normal capillaries, arteries or veins, specifically including glomeruloid, bulbous or angioma-like structures, hemangiomas, and chronically hyperpermeable vessels.

*Heterogeneous population* - is a population of transduced cells, composed of several different clonal subpopulations, each of which expresses a different level of the transgene over a wide range of values.

*Target gene* - is a gene whose gene product requires tight expression control in individual cells transduced with its coding sequences, in order to achieve a desired functional effect, while avoiding toxic effects. Examples include: (1) secreted growth factors/cytokines; (2) membrane and intracellular receptors; and (3) transcription factors. As relating to the process of angiogenesis, examples of each category may include: (1) members of the VEGF/PIGF, PDGF, TGF-beta, or Angiopoietin families; (2) their respective receptors; (3) naturally-occurring or modified, constitutively active Hypoxia Inducible Factor (HIF) molecules (K.A. Vincent et al., Circulation 102:2255-2261, 2000), or angiogenic factors-activating engineered transcription factors (E.J. Rebar et al., Nat. Med. 8:1427-1432, 2002).

*Cell-surface marker* - is a protein which: (1) is located on the cell surface, so that it can be quantified by antibody staining and FACS detection; (2) is the product of a human gene, so that it does not elicit an immune reaction; and (3) does not have a biological function in the stably transduced cells, so that its expression does not interfere with cell expansion in vitro and differentiation in vivo. Examples include the lymphocite-specific molecules CD8 and CD4, whose required partners in the T-Cell Receptor Complex are only expressed in T-lymphocites. To further avoid any biological function, said molecules are modified to truncate them at the junction between the transmembrane and intracytoplasmic domains, so that the intracelluler signal-transduction domains are removed, while the transmembrane and extracellular domains, which are responsible for cell-surface localization and antibody recognition, respectively, are retained.

*Sequence accommodating translation* - is a polynucleotide sequence, positioned between a first and a second coding sequence, which allows translation of both coding sequences from the same transcription unit (a single mRNA molecule). Examples include a variety of naturally-occurring or synthetic Internal Ribosomal Entry Sites (IRES), such as the Encephalomyocarditis virus IRES described in the experimental part hereinbelow. Alternatively, IRES sequences derived from rhinovirus, aphthovirus, cardiovirus, encephalomyocarditis virus, enterovirus, adenovirus, influenza virus, herpes virus cytomegalovirus, HIV, mengovirus, bovine viral diarrhea virus, hepatitis A, B or C virus, GTX, Cyr61 a, Cyr61 b, poliovirus, picornavirus, murine encephalomyocarditis virus, poliovirus, and foot and mouth disease virus may be used. Fragments, mutants and variants of naturally occurring IRES sequences may be employed provided they retain the ability to initiate translation of an operably linked coding sequence located 3' of the IRES.

*Correlate* - means to establish a quantitative relationship between the levels of expression of the target gene and of the cell-surface marker in each transduced cell, by measuring each in clonal populations (in which each cell expresses the same level), so that measurement of the second may give information on the value of the first in individual cells in heterogeneous populations.

The rationale for the inventive method is the following: The distribution of microenvironmental expression levels can be reliably controlled by implanting clonal myoblast populations stably expressing different homogeneous levels of VEGF per cell (C.R. Ozawa, A. Banfi et al., J. Clin. Invest. 113:515-527, 2004). The therapeutic potential of controlling the microenvironmental distribution of VEGF levels is shown in a murine model of hindlimb ischemia. Implantation of a polyclonal myoblast population, expressing VEGF₁₆₄ at 60 ng/10⁶ cells/day on average, increases blood flow only moderately (Fig. 1a), and vascular leakage and aberrant pre-angiomatous vessels are always induced. However, when the same total VEGF dose is uniformly distributed in muscle by implanting a monoclonal population, blood flow is fully restored to nonischemic levels (Fig. 1a), accompanied by arteriole and collateral growth (Fig. 1b) and reduced ischemic tissue damage. Aberrant vasculature and hemangiomas are avoided and only normal, pericyte-covered vessels are induced which persist over 15 months. While providing compelling proof-of-concept of the therapeutic improvement afforded by VEGF gene delivery at controlled levels, this approach is not feasible in clinical practice. In fact, it involves the isolation and characterization of multiple clonal populations of transduced autologous myoblasts, which requires both excessive time and resources for each patient. To make this concept clinically applicable, it is desirable to be able to rapidly purify cells expressing the desired VEGF level from a randomly transduced autologous population, without producing clones for every patient.

In a particular embodiment of the invention, in order to achieve controlled gene delivery to skeletal muscle, primary myoblasts are transduced with a retroviral construct (mVICD8) in which the VEGF₁₆₄ gene is linked through an Internal Ribosomal Entry Site (IRES) to a truncated version of CD8a (murine Ly2 gene, Fig. 2). The intracellular domain of this lymphocyte-specific molecule is removed, such that it retains no biological function and acts purely as a cell-surface reporter gene. An IRES sequence allows ribosome assembly on the mRNA and the initiation of translation in a cap-independent fashion (I.R. Ghattas et al., Mol. Cell. Biol. 11: 5848-5859, 1991). Therefore it allows the translation of two genes from the same mRNA: The first by the classic mechanism and the second through the IRES. In this way, changes in the level of expression of VEGF are reflected by a parallel change in cell-surface expression of CD8a, which can be detected and quantified on live cells by Fluorescence Activated Cell Sorting (FACS).

The basic concept underlying this embodiment of the invention is that the linking of VEGF expression to that of CD8 on the cell surface allows the prediction of the level of expression of VEGF on a cell-by-cell basis by quantification of the CD8 level by FACS. However, this is not possible directly, because the absolute intensity of fluorescence detected by the FACS depends on many variables, including the efficiency of antibody staining, the laser intensity and the settings of the detectors, among others. Therefore, in order to reproducibly identify the cells expressing a desired VEGF level in the heterogeneous primary transduced population, it is necessary to have a reference population which stably and homogeneously expresses that level and which is stained and analyzed together with the primary cells. The cells which co-localize with the reference population on the FACS plot express equivalent VEGF levels and can be sorted to purity.

Primary mouse myoblasts have been transduced with the mVICD8 retroviral construct, generating a very heterogeneous polyclonal population. In order to establish the relationship between VEGF production and level of CD8 expression, single clones have been isolated from this population, stably expressing different and homogeneous VEGF and CD8 levels (Fig. 3).

A set of 8 clones expressing different levels of VEGF were also injected in vivo in the posterior auricular muscle of immunodeficient SCID mice, as previously described (C.R Ozawa, A. Banfi et al., J. Clin. Invest. 113:515-527, 2004), in order to evaluate the induced vascular phenotype. The population that expressed the highest VEGF amount, while at the same time completely avoiding aberrant angiogenesis (-40 ng/10⁶ cells/day) was selected as the reference clone. The stability of VEGF and CD8 expression by this clone was tested and it was found to be constant over at least 26 population doublings.

As shown in Fig. 4, the reference clone was used to identify 2 different polyclonal sub-populations of interest from the primary heterogeneous population. Based on their level of CD8 expression, one group of cells was sorted corresponding to the whole width of the reference clone (large gate B. in Fig. 4) or just to its lower half (narrow gate A.).

The two sorted populations were cultured in vitro to simulate the expansion necessary to achieve sufficient cells for clinical application. Both CD8 expression and VEGF production (-20 and -30 ng/10⁶ cells/day for the narrow and large populations, respectively) were found to be stable over at least 23 populations doublings (Fig. 5). This degree of expansion would yield over 8x10¹³ cells from an initial sort of 1 million, which is several orders of magnitude in excess of the amount needed for clinical delivery (estimated in the range of 10⁹ cells). When implanted in vivo in the posterior auricular muscle of SCID mice, the sorted populations showed that a highly significant enrichment of the cells expressing the desired VEGF levels had been achieved. As shown in Fig. 6, the heterogeneous polyclonal population invariably induced aberrant bulbous structures, which evolve into progressive cavernous hemangiomas, as expected. However, the cells sorted from this same population based on their CD8 expression (both with the narrow and large gates) caused the growth of normal, homogeneously sized capillaries, similarly to the reference clone. Analysis of the vessel diameter distribution shows that normal capillaries have sizes comprised between 5 and 15 µm, with a peak below 10 µm (Fig. 6, control C-). The bulbous structures induced by heterogeneous high levels of VEGF have aberrantly dilated diameters, often larger than 30 µm (Fig. 6, unsorted N.S.). Both sorted populations induce angiogenesis with a normal diameter distribution, similarly to the reference clone (Fig. 6, S1, S2 and Ref.).

Both sorted populations preserve a normal capillary phenotype, but also efficiently induce new vascular growth. In fact, vessel length density (VLD), which measures the total length of vessels in a defined area and is not influenced by diameter, is doubled in the areas implanted with both sorted populations compared to control (Fig. 7, left panel), similarly to the effects of the reference clone. Interestingly, dishomogeneous and high VEGF levels expressed by the unsorted population actually decrease VLD, because they induce hyperfusion of pre-existing vessels and circumferential growth of bulbous structures, rather than sprouting of new capillaries (Fig. 7, left panel).

This increase in VLD represents an actual increase in vessel number and not simply an elongation of pre-existing capillaries. This is shown by the quantification of the branching index, which measures the number of branch points/100 µm of vessel length. As shown in Fig. 7 (right panel), both sorted populations not only increase VLD compared to control, but also the frequency of branch points. Therefore, the resulting vessels, defined as the vascular segments between 2 branch points, are slightly shorter (more branches) and more than double in number compared to controls. Again, these features are completely similar to the effects of implantation of the reference clone.

At 4 weeks post-implantation in the auricular muscle, only normal angiogenesis had been induced in 4 out of 5 animals injected with each of the 2 sorted populations.

As the person skilled in the art will appreciate, variations of the above described protocols are possible. For example, the sorting gate might assume different sizes (the whole size of the reference clone vs. a tighter gate spanning half the width and centred on the peak) and, apart from a single round of sorting, multiple successive rounds of sorting, such as 2 to 3, may be performed. Furthermore, the center of the sorting gate may be positioned on different expression values, precalculated on the basis of a "reference line" obtained from the values of 2 or more different reference clones, so that populations may be obtained which express any desired level of the target gene within the range of the polyclonal population. Precise definition of the minimum required conditions that reproducibly yield homogeneous safe and efficacious populations is accordingly possible.

It should also be noticed that, while the above-described experiments are performed in the field of therapeutic angiogenesis by VEGF gene delivery, this invention is readily applicable to other genes of interest, in particular those for which it is necessary to tightly control expression levels in each individual transduced cell, such as other secreted growth factors, transcription factors and membrane or intracellular receptors.

## Claims

1. A method for producing precursor cells expressing Vascular Endothelial Growth Factor (VEGF) at desired levels comprising
(a) providing a heterogeneous population of cultured precursor cells selected from primary myoblasts, bone marrow derived mesenchymal stem cells, adipose tissue-derived mesenchymal stem cells and cardiac stem cells;
(b) stably transducing said precursor cells with (i) VEGF linked to (ii) a truncated version of CD8/CD8a, translated from the same mRNA via an internal ribosomal entry site (IRES);
(c) correlating an expression level of VEGF with that of the truncated version of CD8/CD8a;
(d) selecting a reference clone of cultured precursor cells expressing VEGF at a threshold of desirability; and
(e) isolating precursor cells having the (i) same or lower levels or (ii) the same or higher levels of expression of VEGF than said reference clone via said truncated version of CD8/CD8a.

2. The method of claim 1 wherein in step (d) two reference clones are selected expressing VEGF at a lower threshold and at an upper threshold, respectively, and precursor cells are isolated in step (e) having a level of expression of VEGF between the levels of expression of the two reference clones.

3. The method of claim 1 wherein in step (d) two or more reference clones are selected expressing VEGF at different levels, said reference clones used to define a lower and an upper threshold of VEGF expression, and precursor cells isolated in step (e) having a level of expression of VEGF at any desired level between the lower and higher threshold levels of expression.

4. The method of claims 1, 2 or 3 wherein the expression level of said truncated version of CD8/CD8a in step (d) is quantified via Fluorescence Activated Cell Sorting (FACS).

5. The method of claim 1, 2 or 3 wherein the expression level of VEGF in step (d) is quantified via a method selected from ELISA, RIA, EIA, Western Blot and Surface Plasmon Resonance.

6. A system for producing precursor cells expressing VEGF in a host at desirable levels, comprising
(a) a construct integrating into the host genome providing (i) VEGF linked to (ii) a truncated version of CD8/CD8a, translated from the same mRNA via an internal ribosomal entry site (IRES); and
(b) instructions for:
(1) stably transducing precursor cells selected from primary myoblasts, bone marrow derived mesenchymal stem cells, adipose tissue-derived mesenchymal stem cells and cardiac stem cells with said construct; and
(2) correlating expression levels of VEGF with that of the truncated version of CD8/CD8a so that expression levels of VEGF can be assessed in said precursor cells via the truncated version of CD8/CD8a.

7. A system according to claim 6 wherein the construct is a retroviral construct.

8. A composition for use in gene therapy comprising
a homogenous population of precursor cells selected from primary myoblasts, bone marrow derived mesenchymal stem cells, adipose tissue-derived mesenchymal stem cells and cardiac stem cells stably transduced with a construct providing (i) VEGF linked to (ii) a truncated version of CD8/CD8a, translated from the same mRNA via an internal ribosomal entry site (IRES), wherein the homogenous population has been selected via said truncated version of CD8/CD8a so that the expression levels of VEGF are as desired in a host microenvironment into which said precursor cells are to be introduced.

## Patentansprüche

1. Verfahren zum Herstellen von Vorläuferzellen, die den vaskulären Endothelwachstumsfaktor (VEGF) in gewünschten Höhen exprimieren, umfassend:
(a) Bereitstellen einer heterogenen Population von gezüchteten Vorläuferzellen, die aus primären Myoblasten, von Knochenmark abgeleiteten mesenchymalen Stammzellen, von Fettgewebe abgeleiteten mesenchymalen Stammzellen und Herzstammzellen ausgewählt sind;
(b) stabiles Transduzieren der Vorläuferzellen mit (i) VEGF, mit (ii) einer gekürzten Version von CD8/CD8a verknüpft, aus der gleichen mRNA über eine interne ribosomale Eintrittsstelle (IRES) translatiert;
(c) Korrelieren einer VEGF-Expressionshöhe mit jener der gekürzten Version von CD8/CD8a;
(d) Auswählen eines Referenzklons von gezüchteten Vorläuferzellen, der VEGF mit einem gewünschten Schwellenwert exprimiert; und
(e) Isolieren von Vorläuferzellen mit (i) den gleichen oder geringeren VEGF-Expressionshöhen oder (ii) den gleichen oder höheren VEGF-Expressionshöhen als der Referenzklon über die gekürzte Version von CD8/CD8a.

2. Verfahren nach Anspruch 1, wobei in Schritt (d) zwei Referenzklone ausgewählt werden, die VEGF mit einem unteren Schwellenwert bzw. einem oberen Schwellenwert exprimieren, und in Schritt (e) Vorläuferzellen mit einer VEGF-Expressionshöhe zwischen den Expressionshöhen der zwei Referenzklone isoliert werden.

3. Verfahren nach Anspruch 1, wobei in Schritt (d) zwei oder mehr Referenzklone ausgewählt werden, die VEGF in unterschiedlichen Höhen exprimieren, wobei die Referenzklone verwendet werden, um einen unteren und einen oberen Schwellenwert der VEGF-Expression zu definieren, und wobei in Schritt (e) Vorläuferzellen mit einer VEGF-Expressionshöhe in einer beliebigen Höhe zwischen dem unteren und dem oberen Schwellenwert der Expressionshöhe isoliert werden.

4. Verfahren nach Anspruch 1, 2 oder 3, wobei die Expressionshöhe der gekürzten Version von CD8/CD8a in Schritt (d) über fluoreszenzaktivierte Zellsortierung (FACS) quantifiziert wird.

5. Verfahren nach Anspruch 1, 2 oder 3, wobei die VEGF-Expressionshöhe in Schritt (d) über ein Verfahren quantifiziert wird, das aus ELISA, RIA, EIA, Western Blot und Oberflächenplasmonenresonanz ausgewählt ist.

6. System zum Herstellen von Vorläuferzellen, die VEGF in einem Wirten in wünschenswerten Höhen exprimieren, umfassend:
(a) ein Konstrukt, das sich in das Wirtsgenom einbindet, das (i) VEGF bereitstellt, mit (ii) einer gekürzten Version von CD8/CD8a verknüpft, aus der gleichen mRNA über eine interne ribosomale Eintrittsstelle (IRES) translatiert; und
(b) Anweisungen zum:
(1) stabilen Transduzieren von Vorläuferzellen, die aus primären Myoblasten, von Knochenmark abgeleiteten mesenchymalen Stammzellen, von Fettgewebe abgeleiteten mesenchymalen Stammzellen und Herzstammzellen ausgewählt sind, mit dem Konstrukt; und
(2) Korrelieren von VEGF-Expressionshöhe mit jener der gekürzten Version von CD8/CD8a, so dass VEGF-Expressionshöhen in den Vorläuferzellen über die gekürzte Version von CD8/CD8a beurteilt werden können.

7. System nach Anspruch 6, wobei das Konstrukt ein retrovirales Konstrukt ist.

8. Zusammensetzung zur Verwendung in der Gentherapie, umfassend:
eine homogene Population von Vorläuferzellen, die aus primären Myoblasten, von Knochenmark abgeleiteten mesenchymalen Stammzellen, von Fettgewebe abgeleiteten mesenchymalen Stammzellen und Herzstammzellen ausgewählt sind, die stabil mit einem Konstrukt transduziert sind, das (i) VEGF bereitstellt, mit (ii) einer gekürzten Version von CD8/CD8a verknüpft, aus der gleichen mRNA über eine interne ribosomale Eintrittsstelle (IRES) translatiert, wobei die homogene Population über die gekürzte Version von CD8/CD8a ausgewählt wurde, so dass die VEGF-Expressionshöhen in einer Wirtsmikroumgebung, in die die Vorläuferzellen einzubringen sind, wie gewünscht sind.

## Revendications

1. Procédé pour produire des cellules précurseurs exprimant le facteur de croissance de l'endothélium vasculaire (VEGF) à des taux souhaités, comprenant
(a) la mise à disposition d'une population hétérogène de cellules précurseurs en culture sélectionnées parmi des myoblastes primaires, des cellules souches mésenchymateuses dérivées de moelle osseuse, des cellules souches mésenchymateuses dérivées de tissu adipeux et des cellules souches cardiaques ;
(b) la transduction stable desdites cellules précurseurs avec (i) le VEGF lié à (ii) une version tronquée de CD8/CD8a, traduite à partir du même ARNm via un site d'entrée interne des ribosomes (IRES) ;
(c) la corrélation d'un taux d'expression du VEGF avec celui de la version tronquée de CD8/CD8a ;
(d) la sélection d'un clone de référence des cellules précurseurs en culture exprimant le VEGF à un seuil souhaitable ; et
(e) l'isolement de cellules précurseurs présentant (i) des taux identiques ou plus faibles ou (ii) des taux identiques ou plus élevés d'expression du VEGF par rapport audit clone de référence via ladite version tronquée de CD8/CD8a.

2. Procédé selon la revendication 1 dans lequel, à l'étape (d), deux clones de référence sont sélectionnés en exprimant le VEGF à un seuil inférieur et à un seuil supérieur, respectivement, et des cellules précurseurs sont isolées à l'étape (e) en présentant un taux d'expression du VEGF compris entre les taux d'expression des deux clones de référence.

3. Procédé selon la revendication 1 dans lequel, à l'étape (d), deux clones de référence ou plus sont sélectionnés en exprimant le VEGF à différents taux, lesdits clones de référence étant utilisés pour définir un seuil d'expression du VEGF inférieur et supérieur, et les cellules précurseurs sont isolées à l'étape (e) en présentant un taux d'expression du VEGF à un quelconque taux souhaité entre les taux d'expression de seuil inférieur et supérieur.

4. Procédé selon les revendications 1, 2 ou 3, dans lequel le taux d'expression de ladite version tronquée de CD8/CD8a à l'étape (d) est quantifié via un tri cellulaire activé par fluorescence (FACS).

5. Procédé selon les revendications 1, 2 ou 3, dans lequel le taux d'expression du VEGF à l'étape (d) est quantifié via un procédé sélectionné parmi un ELISA, RIA, EIA, transfert de Western et résonance plasmonique de surface.

6. Système pour produire des cellules précurseurs exprimant le VEGF dans un hôte à des taux souhaitables, comprenant
(a) un produit de recombinaison qui s'intègre dans le génome de l'hôte fournissant (i) le VEGF lié à (ii) une version tronquée de CD8/CD8a, traduite à partir du même ARNm via un site d'entrée interne des ribosomes (IRES) ; et
(b) des instructions pour :
(1) stablement transduire des cellules précurseurs sélectionnées parmi des myoblastes primaires, des cellules souches mésenchymateuses dérivées de moelle osseuse, des cellules souches mésenchymateuses dérivées de tissu adipeux et des cellules souches cardiaques, avec ledit produit de recombinaison ; et
(2) corréler les taux d'expression du VEGF avec ceux de la version tronquée de CD8/CD8a de sorte que les taux d'expression du VEGF puissent être évalués dans lesdites cellules précurseurs via la version tronquée de CD8/CD8a.

7. Système selon la revendication 6, dans lequel le produit de recombinaison est un produit de recombinaison rétroviral.

8. Composition destinée à être utilisée en thérapie génique comprenant
une population homogène de cellules précurseurs sélectionnées parmi des myoblastes primaires, des cellules souches mésenchymateuses dérivées de moelle osseuse, des cellules souches mésenchymateuses dérivées de tissu adipeux et des cellules souches cardiaques stablement transduites avec un produit de recombinaison fournissant (i) le VEGF lié à (ii) une version tronquée de CD8/CD8a, traduite à partir du même ARNm via un site d'entrée interne des ribosomes (IRES), où la population homogène a été sélectionnée via ladite version tronquée de CD8/CD8a de sorte que les taux d'expression du VEGF soient tel que souhaité dans le microenvironnement d'un hôte dans lequel lesdites cellules précurseurs doivent être introduites.
